# EUROPEAN PATENT APPLICATION

(11) **EP 4 483 796 A1**
(43) Date of publication of application: **01.01.2025**
(21) Application number: 24184256.6
(22) Date of filing: 25.06.2024
(51) Int. Cl.: A61B 5/024, A61B 5/00, G16H 50/20

(54) **METHOD AND DEVICE FOR SIGNAL TRANSLATION**

(30) Priority: 28.06.2023 US 202318343704
(71) Applicant: Analog Devices International Unlimited Company, Limerick (IE)
(72) Inventor: Bolger, Eoin Seamus, Limerick (IE); Dempsey, Dennis, Limerick (IE)
(74) Representative: Horler, Philip John

(57) **Abstract**

A source stochastic signal is deconstructed into one or more components using a decomposition process. The decomposition process deconstructs the source stochastic signal into at least one component which encodes the oscillatory behavior of the source stochastic signal. The one or more components are mapped, using one or more trained models, to a pair of target components which encode the estimated oscillatory behavior and estimated steady-state behavior of a target stochastic signal. The target stochastic signal is then reconstructed from the oscillatory behavior target signal by shifting the oscillatory behavior target signal so that its average value aligns with the average value of the steady-state behavior target signal. The source stochastic signal and the target stochastic signals can be biological signals having a related or common origin, such as photoplethysmogram (PPG) signals and arterial blood pressure (ABP) waveforms.

## Description

### FIELD OF DISCLOSURE

The present disclosure relates to the translation of a first stochastic signal to a second stochastic signal. Particularly but not exclusively, the present disclosure relates to the automatic translation of a first stochastic biological signal to a second stochastic biological signal; more particularly, wherein the first stochastic biological signal is a photoplethysmogram or electrocardiogram signal and the second stochastic biological signal is an arterial blood pressure waveform.

### BACKGROUND

In current clinical practice, blood pressure measurement is performed either invasively using an intra-arterial catheter, or noninvasively by cuff using either oscillometric or auscultatory methods. Invasive measurement is continuous in nature but carries risk (e.g., infection, bleeding, thrombosis). As such, invasive measurement is used only for critically ill patients and not for the majority of patients with chronic hypertension. Intermittent cuff inflation, the gold standard for blood pressure measurement in chronic hypertension, is non-invasive but does not allow for continuous blood pressure measurement.

A photoplethysmogram (PPG) is a non-invasive circulatory signal related to the pulsatile volume of blood in tissue and is typically collected by pulse oximeters. Blood pressure can be estimated from a PPG signal which can be obtained from a variety of devices. Such devices include mobile or wearable devices such as a smart watch, activity tracker, and the like. However, PPG signals are prone to artefacts that can negatively impact measurement accuracy. Such artefacts can lead to a significant number of misleading diagnoses. These artefacts are more pronounced in PPG signals collected by mobile devices due to increased movement of the device. The accuracy of the reading can vary based on factors such as blood flow, device placement, and stillness of the wearer of the device.

Blood pressure estimation is typically approached as a regression problem, whereby features are extracted from a combination of biological signals, which typically include PPG and electrocardiogram (ECG). Features can also include signals obtained from activity or environment sensors such as accelerometers, pressure sensors, blood oxygen sensors, and the like. These features are then input to a machine learning algorithm in order to predict blood pressure. Examples of machine learning algorithms suitable for blood pressure prediction include linear regression, support vector regression, Bayesian regression, and regression based deep neural networks. Within a regression session, these techniques can predict systolic blood pressure (SBP), diastolic blood pressure (DBP), and/or mean arterial pressure (MAP). Classification methods, such as logistic regression, can also be used to predict categorical indicators such as normal, prehypertension, stage one hypertension, and stage two hypertension.

One of the issues with the above methods is that fine features, like the dicrotic notch and the dicrotic peak, are not always present in PPG signals and are difficult to capture reliably. Moreover, existing approaches to mapping between PPG and arterial blood pressure (ABP) signals may capture an approximation of the morphology, or shape, of the ABP signal but fail to reconstruct the absolute values of the ABP signal correctly. Consequently, most existing techniques are unsuitable to be used for the generation of signals from which relevant features, such as the diastolic or systolic blood pressure, are to be extracted since these features rely on accurate reconstruction not only of the signal's morphology, but also of the signal's amplitude values (i.e., the absolute ABP signal values).

### SUMMARY OF DISCLOSURE

In the present disclosure, a source stochastic signal is deconstructed into one or more components using a decomposition process. The decomposition process deconstructs the source stochastic signal into at least one component which encodes the oscillatory behavior of the source stochastic signal. The one or more components are mapped, using one or more trained models, to a pair of target components which encode the estimated oscillatory behavior and estimated steady-state behavior of a target stochastic signal. The target stochastic signal is then reconstructed from the oscillatory behavior target signal by shifting the oscillatory behavior target signal so that its average value aligns with the average value of the steady-state behavior target signal. The source stochastic signal and the target stochastic signals can be biological signals having a related or common origin, such as photoplethysmogram (PPG) signals and arterial blood pressure (ABP) waveforms.

The present disclosure provides a method and device for the translation of stochastic signals, in particular biological signals having a related origin. A source stochastic signal is decomposed into one or more source components. The one or more source components include at least a first component associated with the oscillatory behavior of the source signal. The one or more source components are mapped, using one or more trained machine learning models, to a first target component and a second target component. The first target component comprises an estimate of the oscillatory behavior of a target signal, and the second target component comprises an estimate of the steady-state behavior of the target signal. The target signal is then reconstructed from the first target signal and the second target signal.

As such, aspects of the present disclosure provide accurate reconstruction of the morphology and power (amplitude) of a target stochastic signal from a source stochastic signal. This is particularly advantageous when reconstructing an ABP signal from a PPG signal where both the morphology and power of the ABP signal are needed for accurate extraction of features such as diastolic or systolic blood pressure. When the methods of the present disclosure are used as part of a device for cuff-less blood pressure estimate, more accurate reconstructions of a patient's arterial blood pressure can be obtained. This may help improve patient outcomes whilst also improving the patient's comfort by avoiding the need of a blood pressure cuff to obtain an estimate of ABP. Moreover, embodiments of the present disclosure provide an efficient approach to reconstructing a target stochastic signal from a source stochastic signal using only a single trained model. Because only a single model needs to be trained and held in memory, this may help make more efficient use of storage space and consume less power in use. Both of these advantages may be particularly beneficial for use on low power devices such as wearable devices, activity trackers, or smart watches.

Further features and aspects of the disclosure are provided in the appended claims.

### BRIEF DESCRIPTION OF DRAWINGS

The present disclosure will now be described by way of example only with reference to the accompanying drawings in which:
Figure 1 shows a system for signal reconstruction according to an aspect of the present disclosure;
Figure 2A shows an example photoplethysmogram (PPG) signal according to an embodiment of the present disclosure;
Figure 2B shows an example complex map in the form of a scalogram according to an embodiment of the present disclosure;
Figure 2C shows an example complex map in the form of a spectrogram according to an embodiment of the present disclosure;
Figures 3A and 3B show high-level architectures of U-net neural networks according to embodiments of the present disclosure;
Figures 4A and 4B show encoder and decoder architectures of the U-net neural networks shown in Figures 3A and 3B according to embodiments of the present disclosure;
Figure 5 shows a plot of target signal reconstruction according to an aspect of the present disclosure;
Figure 6 shows a method for signal reconstruction according to an aspect of the present disclosure;
Figure 7 shows a method for signal reconstruction according to an aspect of the present disclosure;
Figure 8 shows an example computing system suitable for carrying out embodiments of the present disclosure; and
Figure 9 shows a wearable device suitable for carrying out embodiments of the present disclosure.

### DETAILED DESCRIPTION

Many applications within the domain of signal processing involve stochastic signals having a related or common origin. The present disclosure is primarily related to the translation of biological stochastic signals for the purpose of cuff-less blood pressure prediction. Using a combination of signal processing and machine learning, non-invasive photoplethysmogram (PPG) signals are translated to continuous arterial blood pressure (ABP) waveforms. Such ABP waveforms can be used for the further estimation of systolic, diastolic, and central blood pressure values. Whilst sensors for PPG signals are relatively ubiquitous, ABP sensors are typically not present in many at-home devices. The present disclosure therefore addresses the need for at-home, continuous, and non-invasive blood pressure monitoring that is reliable across population variations. Whilst some prior art techniques address this problem using PPG and electrocardiogram (ECG) signals, the present disclosure reconstructs both the morphology and power of an ABP signal from a single PPG signal.

The present disclosure decomposes a source signal into at least one source component which encodes the oscillatory behavior of the source signal. One or more trained machine learning models are used to map from the at least one source component to two target components which encode the estimated oscillatory and steady-state behavior of a target signal. Beneficially, by reconstructing the target signal from the two target components, the morphology and energy of the target signal are accurately reconstructed thereby allowing more accurate features to be extracted from the target signal. By improving the accuracy of features extracted from target signals corresponding to biological readouts such as blood pressure, improvements to patient diagnoses and outcomes can be efficiently made. More generally, the present disclosure not only improves the accuracy of the reconstruction of the target signal, but also does so whilst making efficient use of compute resources and power.

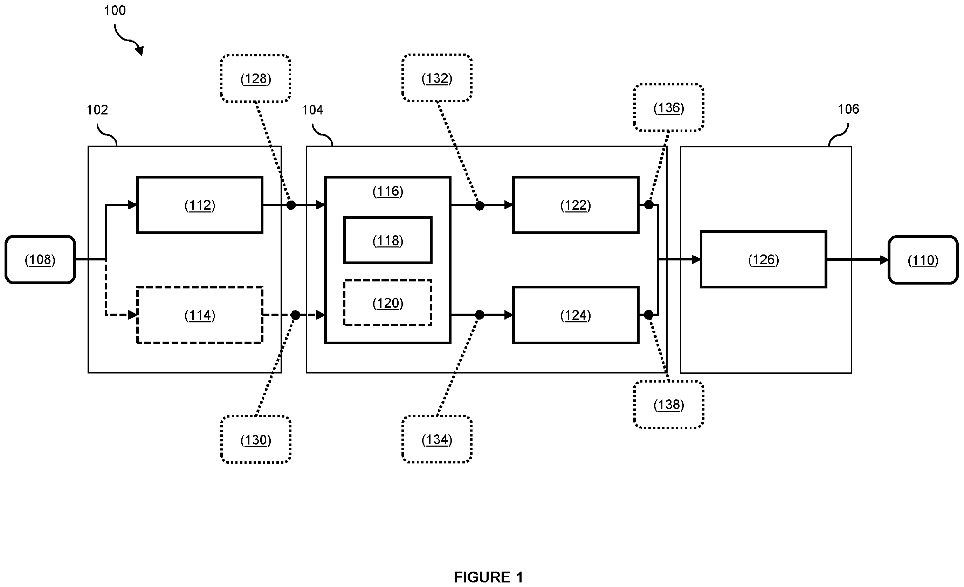

One aspect according to the present disclosure is shown in Figure 1. Figure 1 shows a system 100 for signal reconstruction. The system 100 comprises a deconstruction phase 102, a mapping phase 104, and a reconstruction phase 106. A source signal 108 is provided to the deconstruction phase 102 and a target signal 110 is output from the reconstruction phase 106. The deconstruction phase 102 comprises a first transformation process 112 and optionally comprises a second transformation process 114. The mapping phase 104 comprises one or more trained models 116 which include a first trained model 118 and optionally includes a second trained model 120. The mapping phase 104 further comprises a first inverse transformation process 122 and a second inverse transformation process 124. The reconstruction phase 106 comprises a reconstruction process 126.

The first transformation process 112 transforms the source signal 108 into a first source component 128 whilst the second transformation process 114 transforms the source signal 108 into a second source component 130. The one or more trained models 116 map the component(s) of the source signal 108 to a first target component 132 and a second target component 134. In one embodiment, the one or more trained models 116 map the first source component 128 to the first target component 132 and map the second source component 130 to the second target component 134. In an alternative embodiment, the one or more trained models 116 map the first source component 128 to both the first target component 132 and the second target component 134. The first inverse transformation process 122 applies an inverse transformation to the first target component 132 to generate a first inverse transformed target component 136. The second inverse transformation process 124 applies an inverse transformation to the second target component 134 to generate a second inverse transformed target component 138. The reconstruction process 126 reconstructs the target signal 110 from the first inverse transformed target component 136 and the second inverse transformed target component 138.

Whilst the present disclosure is applicable to the translation of any suitable stochastic signals having a related origin, the present disclosure is primarily directed to biological signals having a related or common origin, and more particularly to biological signals for cuff-less blood pressure prediction. In such settings, the source stochastic signal is a PPG signal and the target stochastic signal is an ABP signal. In this instance, both the PPG signal and the ABP signal have a common origin in the movement and functioning of the heart to which the signals relate.

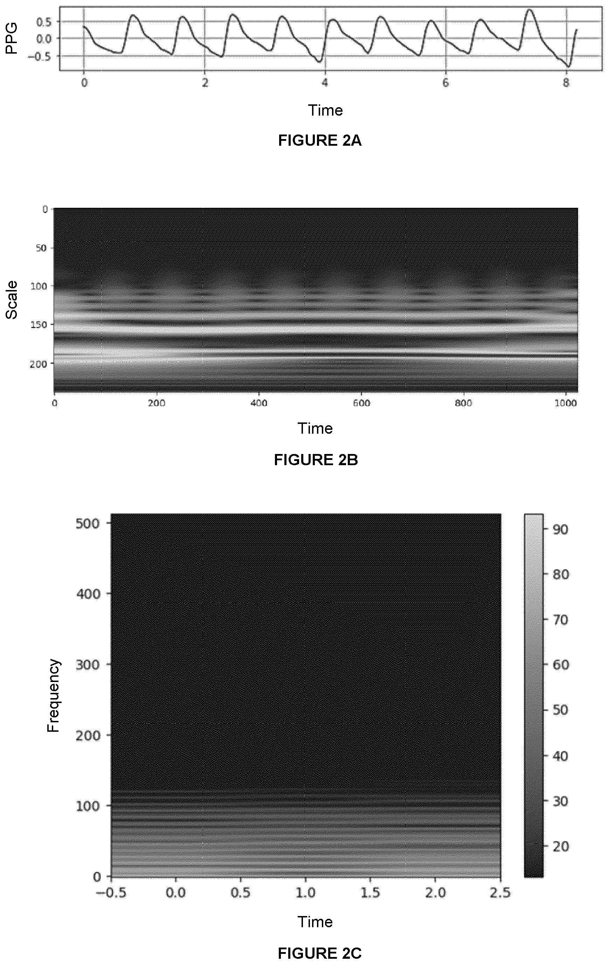

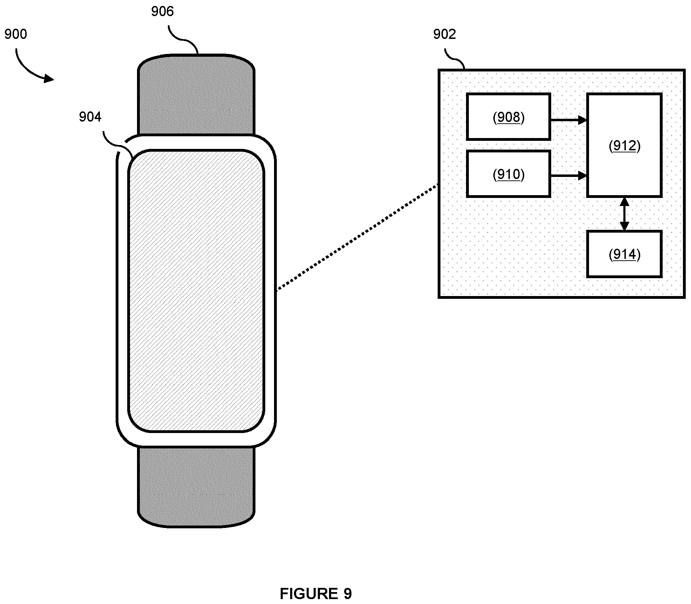

The source signal 108 comprises a signal (e.g., a stochastic signal) of predetermined length obtained from an input signal (not shown). For example, the source signal 108 may be a signal having a length of 10s obtained from an input signal comprising a continuous stream of waveform data (or time-series data) obtained from a sensor such as a photoplethysmogram (PPG) sensor or an electrocardiogram (ECG) sensor. In one embodiment, the source signal 108 is a PPG signal obtained from a PPG sensor of a device such as a cuff-less blood pressure device (e.g., as shown in Figure 9). Figure 2A shows an example PPG signal.

The operations performed during the deconstruction phase 102 decompose the source signal 108 into one or more components which maintain (or capture) at least the oscillatory behavior and may also maintain (or capture) the steady-state behavior of the source signal 108. Here, the oscillatory behavior, or varying behavior, of a signal is understood as the periodic change in direction and magnitude of the signal over time. As such, the oscillatory behavior captured in one of the two components generated during the deconstruction phase 102 may be understood as capturing, or encoding, the alternating current (AC) component of the source signal 108. In contrast, the steady-state behavior of a signal is understood as representing a constant value such that the steady-state behavior captured in one of the two components generated during the deconstruction phase 102 may be understood as capturing, or encoding, the direct current (DC) component the source signal 108. In general, the component encoding the oscillatory behavior of the source signal 108 carries, or encodes, the component of the source signal 108 which varies whilst the component encoding the steady-state behavior of the source signal 108 carries, or encodes, the bias or reference level of the source signal 108. Put another way, one component encodes the morphology of the signal (i.e., the oscillatory behavior) whilst the other component encodes the energy of the signal (i.e., the steady-state behavior). As will be described in more detail below, decomposing the source signal 108 into such components, and reconstructing the target signal 110 from estimates of corresponding components of the target signal 110, provides an efficient and accurate approach for reconstructing the target signal 110. This may be particularly advantageous in healthcare settings where the target signal is a biological signal from which further features are extracted.

The first transformation process 112 of the deconstruction phase 102 decomposes, or transforms, the source signal 108 into the first source component 128 which encodes the oscillatory behavior of the source signal 108. Any suitable transformation process may be used to transform the source signal 108 into the first source component 128. In one embodiment, a continuous wavelet transform (CWT) is used for the first transformation process 112. The CWT analyzes a signal in both the time and frequency domain and captures the oscillatory, or varying, characteristics of the signal (i.e., the AC component of the signal). The signal is convolved with a family of scaled and translated wavelet functions which are derived from a mother wavelet. As such, the CWT decomposes the signal into constituent wavelet components which may be represented as a complex map in the form of a scalogram. An example scalogram is shown in Figure 2B, which shows a 2-dimensional plot, or complex map, obtained from a CWT of a source signal, where the x-axis represents time and the y-axis represents the scale.

The second transformation process 114 of the deconstruction phase 102 in Figure 1 decomposes, or transforms, the source signal 108 into the second source component 130 which encodes the steady-state behavior of the source signal 108. Any suitable transformation process may be used to transform the source signal 108 into the second source component 130. In one embodiment, a Fourier transform is used for the second transformation process 114. The Fourier transform converts a time-domain signal into a frequency-domain representation by decomposing the signal into a sum of sinusoidal components, each having frequency, amplitude, and phase. The Fourier transform may thus be used to capture the steady-state, or constant, characteristic of the signal (i.e., the DC component of the signal). The Fourier transform may be calculated using a Fast Fourier Transform (FFT) algorithm which generates a complex map in the form of a spectrogram from an input signal. An example spectrogram is shown in Figure 2C, which shows a 2-dimensional plot, or complex map, obtained from an FFT of a source signal, where the x-axis represents time and the y-axis represents frequency.

The mapping phase 104 shown in Figure 1 uses the one or more trained models 116 to map from the component(s) of the source signal 108 to target components of the target signal 110. In one embodiment, the one or more trained models 116 are trained to map source components to corresponding target components. That is, the one or more trained models 116 map from a source component which encodes the oscillatory behavior of the source signal 108 (e.g., the first source component 128) to a target component which encodes the (estimated) oscillatory behavior of the target signal 110 (e.g., the first target component 132). Similarly, the one or more trained models 116 map from a source component which encodes the steady-state behavior of the source signal 108 (e.g., the second source component 130) to a target component which encodes the (estimated) steady-state behavior of the target signal 110 (e.g., the second target component 134).

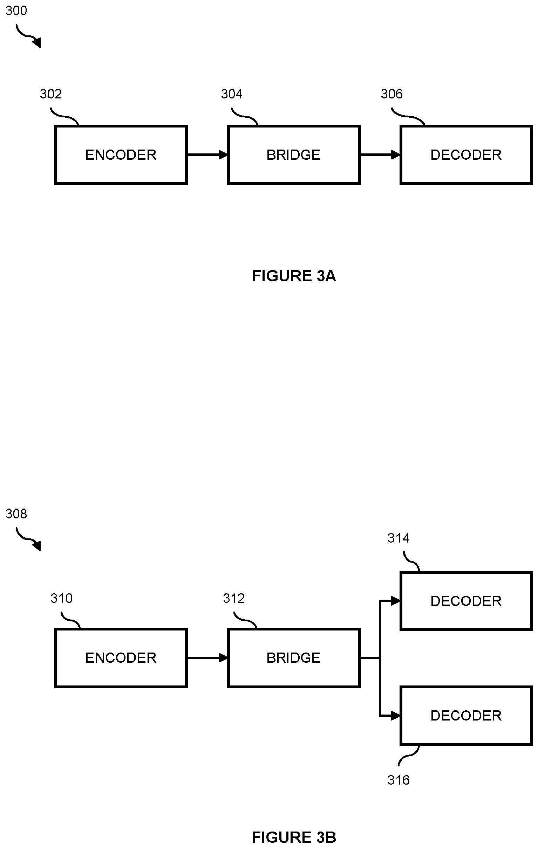

In the presently described embodiment, the one or more trained models 116 include a trained model for each mapping (i.e., a first trained model maps from the first source component 128 to the first target component 132 and a second trained model maps from the second source component 130 to the second target component 134). Each of the trained models correspond to a suitable machine learning model or neural network trained to predict a target component from a source component. Suitable neural network architectures include Language Translation Networks, Recurrent Neural Networks, Convolutional Neural Networks, and the like. In one example, the first trained model and the second trained model are trained encoder-decoder neural networks such as trained U-net models or trained VGG models. An example U-net architecture suitable for use with the present disclosure is shown in Figure 3A.

Figure 3A shows a high-level architecture of a U-net neural network architecture according to an embodiment of the present disclosure.

The U-net architecture 300 shown in Figure 3A comprises an encoder network 302, a bridge 304, and a decoder network 306. The U-net architecture 300 is based on the VGG-16 architecture and uses a dedicated decoder network-the decoder network 306-as opposed to a minimal decoder as used in a fully convolutional network (FCN). U-nets utilize un-pooling layers to recover spatial information and make extensive use of skip connections. These connections make use of spatial information in the encoding path and concatenations of feature maps. As will be described in more detail below, the U-net architecture 300 is trained to map from a source component (e.g., a scalogram or spectrogram associated with a source signal) to an estimated target component (e.g., a scalogram or spectrogram associated with a target signal).

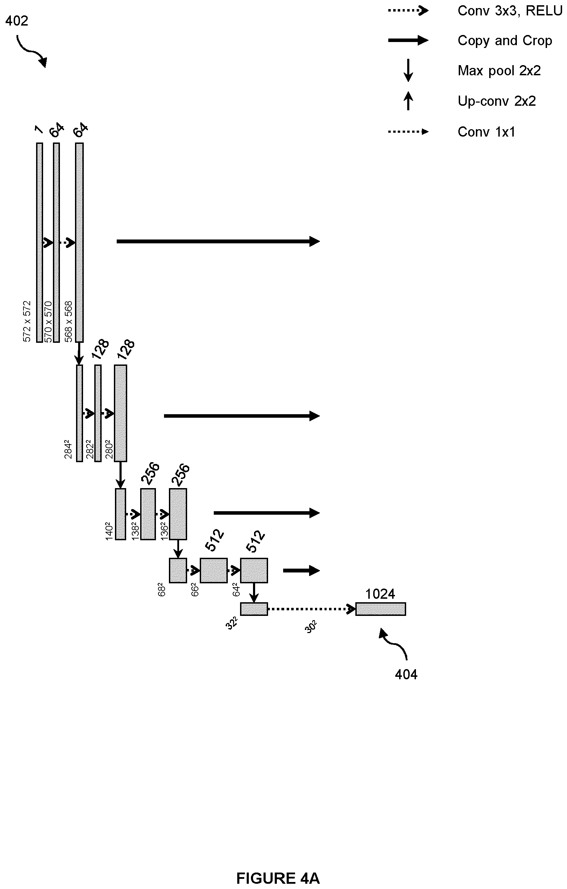

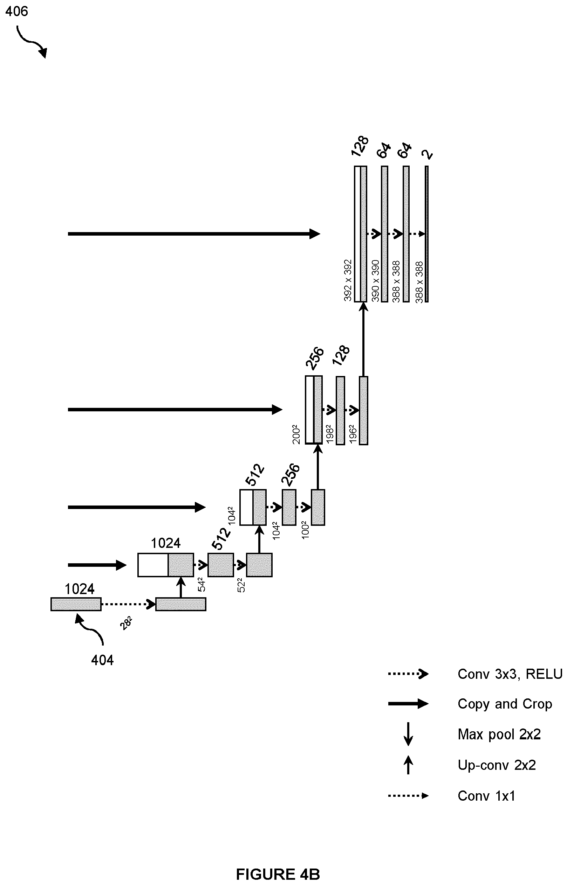

Referring once again to Figure 1, in the presently described embodiment, the one or more trained models 116 shown in Figure 1 comprise one trained U-net model (e.g., according to the U-net architecture 300 shown in Figure 3A) for each of the two signal components. That is, the first trained model 118 may correspond to a first U-net model trained to map from the first source component 128 (e.g., a source scalogram) to the first target component 132 (e.g., a target scalogram) whilst the second trained model 120 may correspond to a second U-net model trained to map from the second source component 130 (e.g., a source spectrogram) to the second target component 134 (e.g., a target spectrogram). Further description of the architecture and training of the U-net models is provided below in relation to Figures 4A and 4B.

In an alternative embodiment, the one or more trained models 116 comprise a single trained model which is configured to map from a source component (e.g., the first source component 128 or the second source component 130) to both target components (e.g., the first target component 132 and the second target component 134). More particularly, the trained model is trained to map from a single complex map-e.g., a scalogram-to a pair of complex maps-a scalogram and a spectrogram. Beneficially, this helps to make more efficient use of compute resources and power which may be particularly advantageous when the present disclosure is implemented on smaller devices where power is limited such as wearable devices, activity trackers, or smart watches. An example single trained model architecture is shown in Figure 3B.

Figure 3B shows a high-level architecture of a U-net neural network architecture for mapping a single source component to two target components according to an embodiment of the present disclosure.

The U-net architecture 308 shown in Figure 3B comprises an encoder network 310, a bridge 312, a first decoder network 314, and a second decoder network 316. The encoder network 310 is configured to receive a source signal component (i.e., a scalogram) as input, the first decoder network 314 is configured to output a first target signal component (e.g., a scalogram), and the second decoder network 316 is configured to output a second target signal component (e.g., a spectrogram).

Having described the general architecture of the example U-net neural networks in Figure 3A and 3B, Figures 4A and 4B show a more detailed architecture of the components of these networks.

Figure 4A shows an encoder architecture 402 for the U-net models shown in Figures 3A and 3B according to an embodiment of the present disclosure.

Figure 4A shows the encoder architecture 402 and a bridge 404. The encoder architecture 402 may correspond to the architecture of the encoder network 302 shown in Figure 3A and/or the architecture of the encoder network 310 shown in Figure 3B. The bridge 404 may correspond to the bridge 304 shown in Figure 3B and/or the bridge 312 shown in Figure 3B. Each of the copy and crop connections shown in Figure 4A are associated with the corresponding copy and crop connections shown in Figure 4B.

Figure 4B shows a decoder architecture 406 for the U-net models shown in Figures 3A and 4B according to an embodiment of the present disclosure.

Figure 4B shows the decoder architecture and the bridge 404 which corresponds to the bridge 404 shown in Figure 4A. The bridge 404 is replicated in both Figure 4A and 4B to provide a common reference between the encoder and decoder architectures. The decoder architecture 406 may correspond to the architecture of the decoder network 306 shown in Figure 3A. Similarly, the decoder architecture 406 may correspond to the architecture of the first decoder network 314 and the second decoder network 316 shown in Figure 3B.

Referring once again to Figure 1, the one or more trained models 116 are trained using a suitable training data set comprising PPG signals with corresponding ABP signals. In one implementation, the one or more trained models 116 are trained using the MIMIC-III database. The MIMIC-III database is a large, freely available database comprising deidentified health-related data associated with over forty thousand patients who stayed in critical care units of the Beth Israel Deaconess Medical Center between 2001 and 2021. The trained models 116 are trained on the MIMIC-III Waveform Database Matched Subset, which contains 22,317 waveform records, and 22,247 numeric records, for 10,282 distinct ICU patients. These recordings typically include digitised signals such as ECG, ABP, respiration, and PPG. The recordings also contain measurements such as heart rate, oxygen saturation, and systolic, mean, and diastolic blood pressure. The database is a subset of the MIMIC-III Waveform Database, representing those records for which the patient has been identified, and their corresponding clinical records are available in the MIMIC-III Clinical Database. The training data taken from the MIMIC-III Waveform Database Matched Subset includes training instances corresponding to PPG signals and training targets corresponding to ABP signals. Both the training instances and the training targets comprise 14,896 waveforms of length 1024.

In embodiments where two trained models are used to map between source components (i.e., the architecture described above in relationship to Figure 3A), the training instances and training targets are decomposed into scalograms and spectrograms. A first of the models is then trained to map from a source scalogram to a target scalogram and a second of the models is trained to map from a source spectrogram to a target spectrogram. In embodiments where a single trained model is used (i.e., the architecture described above n relationship to Figure 3B), the training instances are decomposed into scalograms, and training targets are decomposed into scalograms and spectrograms. The models is then trained to map from a source scalogram to a target scalogram and a target spectrogram. In both of the above mentioned embodiments, the models (the U-net neural networks) are trained using a mean absolute error loss using the ADAM optimizer. The training process is performed over 100 epochs with a batch size of 255 (with shuffling) and 30% of the training data being used for validation.

Once trained, the one or more trained models 116 generate estimated target components of the target signal 110. Because the outputs of the one or more trained models 116 correspond to complex maps (i.e., images), these images are converted to signals prior to the signal being reconstructed. The first inverse transformation process 122 recovers the first inverse transformed target component 136 (i.e., signal) from the first target component 132 (i.e., complex map). That is, the first inverse transformation process 122 recovers a signal, or waveform, from the complex map-i.e., the scalogram-generated by the one or more trained models 116. The inverse transformation performed by the first inverse transformation process 122 corresponds to an inverse of the transformation performed by the first transformation process 112. As such, when a continuous wavelet transform (CWT) is used by the first transformation process 112, then an inverse CWT is used by the first inverse transformation process 122. The second inverse transformation process 124 recovers the second inverse transformed target component 138 (i.e., signal) from the second target component 134 (i.e., complex map). That is, the second inverse transformation process 124 recovers a signal, or waveform, from the complex map-i.e., the spectrogram-generated by the one or more trained models 116. The inverse transformation performed by the second inverse transformation process 124 corresponds to an inverse of the transformation performed by the second transformation process 114. As such, a phase reconstruction algorithm is used by the second inverse transformation process 124 (because there is no direct inverse of the Fourier transform available). In one implementation, the Griffin Lim algorithm is used as the phase reconstruction algorithm.

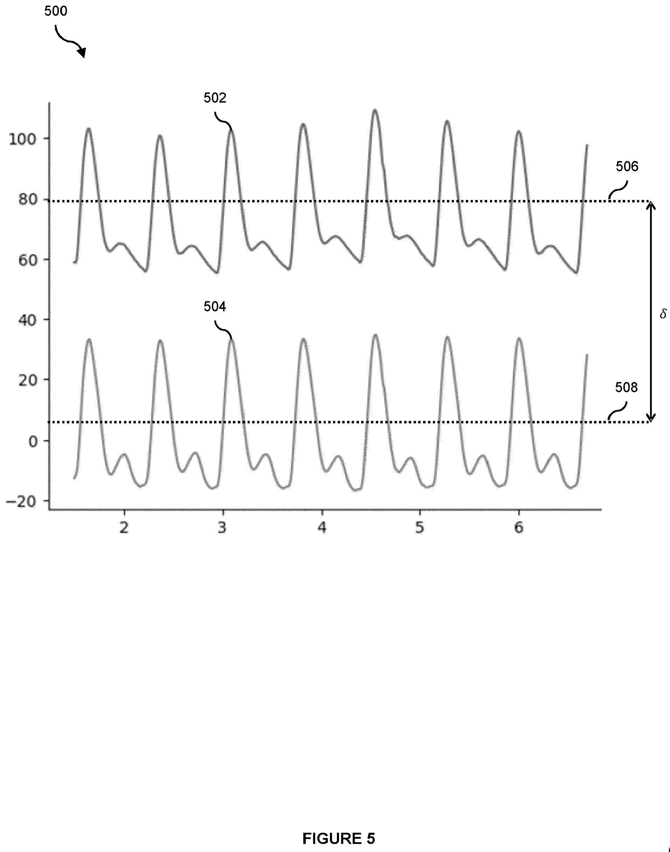

The first inverse transformed target component 136 corresponds to the estimated oscillatory behavior of the target signal 110. That is, the first inverse transformed target component 136 comprises the estimated AC component of the target signal 110. Conversely, the second inverse transformed target component 138 corresponds to the estimated steady-state behavior of the target signal 110. That is, the second inverse transformed target component 138 comprises the estimated DC component of the target signal 110. Whilst the first inverse transformed target component 136 accurately captures the morphology of the target signal, the bias, or reference level, of the signal may not directly correspond to the bias, or reference level, of the target signal. This is shown in Figure 5.

Figure 5 shows a plot 500 of a known target signal 502, an estimated target component 504, a first average value 506, and a second average value 508. The known target signal 502 corresponds to a target signal which is a priori known because it is, for example, a training target which is included in a training dataset. The estimated target component 504 corresponds to a signal obtained via an inverse transformation of a corresponding complex map. More particularly, the estimated target component 504 corresponds to the estimated oscillatory behavior of the target signal obtained from an inverse transformation of a scalogram produced by a respective trained model. The first average value 506 corresponds to the average value of a second estimated target component (not shown) which corresponds to the estimated steady-state behavior of the target signal. The first average value 506 is thus obtained from the signal produced as a result of applying an inverse transformation to a complex map (i.e., spectrogram) produced by a respective trained model. The second average value 508 corresponds to the average of the estimated target component 504.

As can be seen, the estimated target component 504-i.e., the estimated AC component of the target signal-accurately captures the morphology of the known target signal 502, but the bias, or reference levels, of the two signals (i.e., the amplitude values or energy) do not match. Consequently, features such as diastolic blood pressure and systolic blood pressure may be inaccurately reported because they are typically determined from the values of the peaks and troughs of the target (ABP) signal. According to an aspect of the present disclosure, the first average value 506-i.e., the average of the estimated DC component of the target signal-is used to determine an offset, *δ*, which transforms the estimated target component 504 such that its average value (i.e., the average value of the transformed, or offset, inverse transformed target component) matches that of the first average value 506. The offset, *δ*, thus acts to align the estimated AC component of the target signal with the estimated DC component of the target signal thereby accurately reconstructing the target signal. This improvement in accuracy may lead to more accurate and clinically relevant features being extracted from the target signal. As such, the present disclosure may provide improved blood pressure monitoring devices which help improve patient outcomes by more accurately reporting features obtained from the target signal.

Referring once again to Figure 1, the reconstruction process 126 of the reconstruction phase 106 utilizes the approach described in relation to Figure 5 to reconstruct the target signal 110 from the first inverse transformed target component 136 and the second inverse transformed target component 138. That is, the reconstruction process 126 applies a transformation, or translation, to the first inverse transformed target component 136 such that the average (e.g., the mean or median) of the transformed, or translated, first inverse transformed target component is aligned with (or matches) the average (e.g., the mean or median) of the second inverse transformed target component 138. The transformation, or translation, applied by the reconstruction process 126 corresponds to the difference of averages between the first inverse transformed target component 136 and the second inverse transformed target component 138. The target signal 110 thus corresponds to the first inverse transformed target component 136 moved, or translated, such that its average signal value matches the average signal value of the second inverse transformed target component 138. As described above, the operations performed by the reconstruction process 126 may be understood as reconstructing the target signal by aligning the estimated AC component of the signal (i.e., the first inverse transformed target component 136) with the estimated DC component of the signal (i.e., the second inverse transformed target component 138).

In one embodiment, one or more features are extracted from the target signal 110. The one or more features include an estimated diastolic blood pressure and an estimated systolic blood pressure. The estimated diastolic blood pressure is determined from the troughs of the target signal 110. More particularly, a peak detection algorithm is used to identify one or more troughs (one or more local minima which corresponds to the minimum amplitude or value of the target signal at a specific time point) within the target signal 110. The average value of the one or more troughs-for example, the mean or median value-may then be reported, along with the standard deviation, as the estimated diastolic blood pressure. The estimated systolic blood pressure is determined from the peaks of the target signal 110. More particularly, a peak detection algorithm is used to identify one or more peaks (one or more local maxima which corresponds to the maximum amplitude or value of the target signal at a specific time point) within the target signal 110. The average value of the one or more peaks-for example, the mean or median value-may then be reported, along with the standard deviation, as the estimated systolic blood pressure. The one or more features, along with a graphical representation of the target signal 110, may then be output for review by a user.

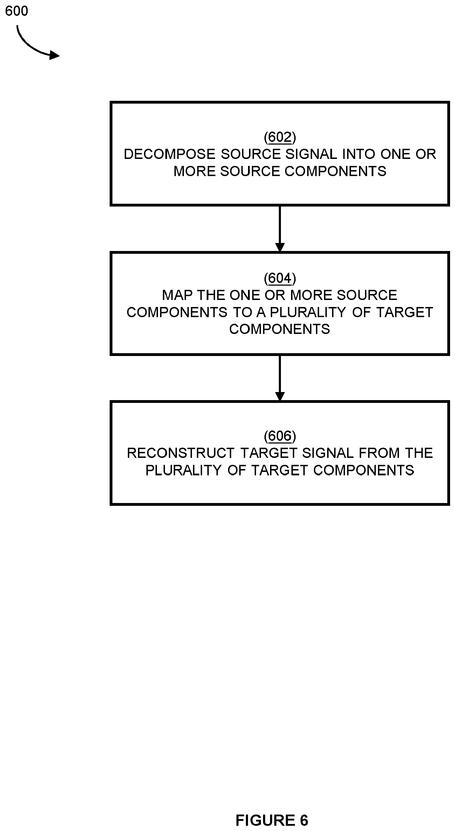

Figure 6 shows a method 600 for signal reconstruction according to an aspect of the present disclosure.

The method 600 comprises the steps of decomposing 602 a source signal into one or more source components, mapping 604 the one or more source components to a plurality of target components, and reconstructing 606 a target signal from the plurality of target components.

In general, the method 600 provides a method for accurately reconstructing the morphology and energy of a target signal from a source signal. By decomposing the source signal into signals which encode the oscillatory and steady state behavior of the source signal, and reconstructing the oscillatory and steady state behavior of the target signal from these components of the source signal, a more accurate reconstruction of the target signal may be obtained. This is particularly advantageous in settings where the accurate morphology and energy values of a target signal are feature extraction (e.g., within a clinical setting where blood pressure values are determined from the target signal). By improving the accuracy of the reconstruction of the target signals, and the subsequent features extracted therefrom, improvements to downstream tasks which utilize such signals and/or features may be achieved.

At the step of decomposing 602, a source signal is decomposed into one or more source components. The one or more source components comprise at least a first source component corresponding to an AC component (oscillatory, or varying, component) of the source signal. As shown in Figure 1, the source signal 108 is decomposed during the deconstruction phase 102 into at least the first source component 128. As described in detail above, the first source component 128 encodes, or captures, the oscillatory behavior-i.e., the AC component-of the source signal 108.

The source signal comprises a signal (e.g., a stochastic signal) of predetermined length obtained from an input signal (not shown). For example, the source signal may be obtained from a (continuous) time-series output of a sensor such as a photoplethysmogram (PPG) sensor or an electrocardiogram (ECG) sensor. In one embodiment, the source signal 108 is a PPG signal, such as that shown in Figure 2A, obtained from a PPG sensor of a device such as a cuff-less blood pressure device (e.g., as shown in Figure 9).

In one embodiment, the source signal is further decomposed into a second source component corresponding to a DC component (steady-state, or constant component) of the source signal. As shown in Figure 1, the source signal 108 may be decomposed during the deconstruction phase 102 into the second source component 130 which encodes, or captures, the steady-state behavior-i.e., the DC component-of the source signal 108.

As stated in more detail above, the source signal is decomposed using one or more transformation processes such as a continuous wavelet transform (CWT) to generate the first source component (AC component) and a Fourier transform to generate the second source component (DC component). Thus, the one or more source components correspond to one or more complex maps which include at least a scalogram associated with the AC component of the source signal and may include a spectrogram associated with the DC component of the source signal.

At the step of mapping 604, the one or more source components are mapped to a first target component and a second target component using one or more trained models. The first target component encodes the estimated oscillatory behavior of the target signal (i.e., the target AC component which is the estimated AC component of the target signal) and the second target component encodes the estimated steady-state behavior of the target signal (i.e., the target DC component which is the estimated DC component of the target signal).

The one or more trained models include one or more trained machine learning models such as one or more trained neural networks. In one embodiment, the one or more trained models comprises a convolutional neural network having a shared encoder and at least two decoders (as shown in Figure 3B). Each of the shared decoders are associated with a respective one of the first target component (the target AC component) or the second target component (the target DC component). In such an embodiment, the first source component (the source AC component, or scalogram) is mapped to the first target component (the target AC component) and the second target component (the target DC component) using a single trained model. In an alternative embodiment, the one or more trained models comprise a first trained model configured to map from the first source component to the first target component and a second trained model configured to map from the second source component to the second target component. An example architecture for the first or second trained model is shown in Figure 3A above.

At the step of reconstructing 606, the target signal is reconstructed from the first target component and the second target component. The reconstructed target signal generated at the step of reconstructing 606 corresponds to a transformed first target component (i.e., a transformed target AC component). The first target component is transformed such that a first average value determined from the transformed first target component (i.e., the transformed target AC component) matches a second average value determined from the second target component (i.e., the transformed target DC component). In embodiments where the source signal corresponds to a PPG signal, the target signal generated at the step of reconstructing 606 corresponds to an estimated ABP signal (i.e., an approximate ABP signal for the user from whom the source PPG signal was obtained).

As shown in Figure 5 and described above, the target signal is reconstructed from the first target component by shifting, or offsetting, the first target component such that it is aligned with the second target component. Put another way, the AC component of the estimated target signal is aligned with the DC component of the estimated target signal thereby reconstructing the target signal from its estimated morphology and energy properties.

In one embodiment, the method 600 further comprises, prior to the step of reconstructing 606, the step of inverse transforming (not shown) the first target component and the second target component using a first inverse transformation process and a second inverse transformation process respectively. The first inverse transformation process and the second inverse transformation process are inverses of the first transformation process and the second transformation process respectively. The target signal is then reconstructed at the step of reconstructing 606 from the inverse transformed representations of the first target component and the second target component.

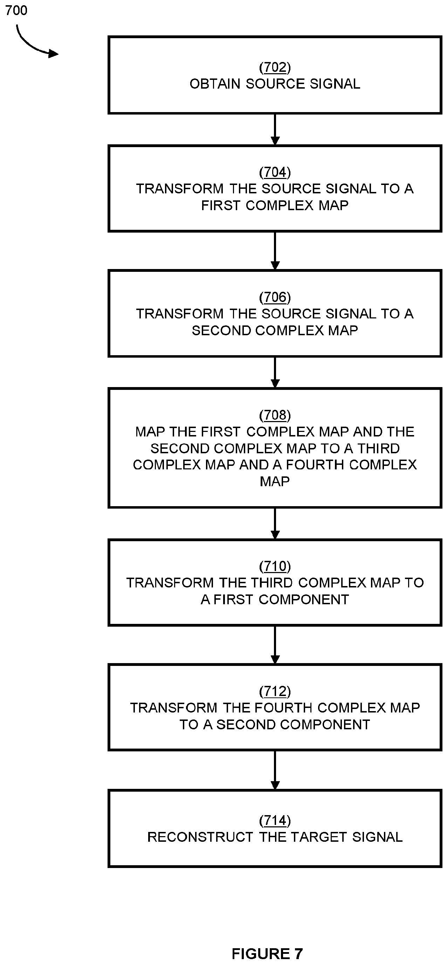

Figure 7 shows a method 700 for signal reconstruction according to an aspect of the present disclosure.

The method 700 comprises the steps of obtaining 702 a source signal, transforming 704 the source signal to a first complex map, transforming 706 the source signal to a second complex map, mapping 708 the first complex map and the second complex map to a third complex map and a fourth complex map respectively, transforming 710 the third complex map to a first component, transforming 712 the fourth complex map to a second component, and reconstructing 714 the target signal from the first component and the second component.

In general, the method 600 provides a method for accurately reconstructing the morphology and energy of a target signal from a source signal. By decomposing the source signal into complex maps which encode behavior of the source signal, and reconstructing the behavior of the target signal from these complex maps, a more accurate reconstruction of the target signal may be obtained. This is particularly advantageous in settings where the accurate morphology and energy values of a target signal are feature extraction (e.g., within a clinical setting where blood pressure values are determined from the target signal). By improving the accuracy of the reconstruction of the target signals, and the subsequent features extracted therefrom, improvements to downstream tasks which utilize such signals and/or features may be achieved.

At the step of obtaining 702, a source stochastic signal is obtained. The source signal comprises a signal (e.g., a stochastic signal) of predetermined length obtained from an input signal (not shown). For example, the source signal may be obtained from a (continuous) time-series output of a sensor such as a photoplethysmogram (PPG) sensor or an electrocardiogram (ECG) sensor. In one embodiment, the source signal 108 is a PPG signal, such as that shown in Figure 2A, obtained from a PPG sensor of a device such as a cuff-less blood pressure device (e.g., as shown in Figure 9).

At the step of transforming 704, the source stochastic signal is transformed to a first complex map using a first transformation process. The first complex map encodes an oscillatory behavior of the source stochastic signal.

The first complex map captures the varying, or oscillatory, characteristics of the source signal and may thus be considered to correspond to the AC component of the source signal. In one embodiment, the first complex map is a scalogram (e.g., as shown in Figure 2B) and the first transformation process is a continuous wavelet transform (CWT).

At the step of transforming 706, the source stochastic signal is transformed to a second complex map using a second transformation process. The second complex map encodes a steady-state behavior of the source stochastic signal.

The second complex map captures the constant, or steady-state, characteristics of the source signal and may thus be considered to correspond to the DC component of the source signal. In one embodiment, the second complex map is a spectrogram (e.g., as shown in Figure 2C) and the second transformation process is a Fourier transform.

At the step of mapping 708, the first complex map and the second complex map are mapped to a third complex map and a fourth complex map respectively using one or more neural networks. The third complex map and the fourth complex map are associated with a target stochastic signal.

The first complex map is mapped to the third complex map using a first trained neural network and the second complex map is mapped to the third complex map using a second trained neural network. As stated above, each of the trained neural networks may comprise a U-net architecture comprising an encoder network, a bridge, and a decoder network (e.g., as shown in Figure 3A).

More details regarding the architecture and training of such neural networks are provided in relation to Figures 4A and 4B above.

At the step of transforming 710, the third complex map is transformed to a first component of the target stochastic signal using a first inverse transformation process associated with the first transformation process. The first component of the target stochastic signal represents the oscillatory behavior of the target stochastic signal.

In one embodiment, the first inverse transformation process is an inverse CWT which corresponds to an inverse of the CWT of the first transformation process.

At the step of transforming 712, the fourth complex map is transformed to a second component of the target stochastic signal using a second inverse transformation process associated with the second transformation process. The second component of the target stochastic signal represents the steady-state behavior of the target stochastic signal.

In one embodiment, the second inverse transformation process is a phase reconstruction algorithm (e.g., the Griffin Lim algorithm) which corresponds to an estimated inverse of the Fourier transform of the second transformation process.

At the step of reconstructing 714, the target stochastic signal is reconstructed from the first component of the target stochastic signal and the second component of the target stochastic signal.

The target stochastic signal is reconstructed by aligning the average of the first component of the target stochastic signal with the average of the second component of the target stochastic signal. As shown in Figure 5, an offset is determined between the two average signal values such that the first component of the target stochastic signal shifted according to the offset generates, or reconstructs, the target stochastic signal.

Optionally, the method 700 further comprises the step of outputting (not shown) the target stochastic signal. For example, the target stochastic signal may be output for viewing by a user, or output to another process or device for further analysis or processing.

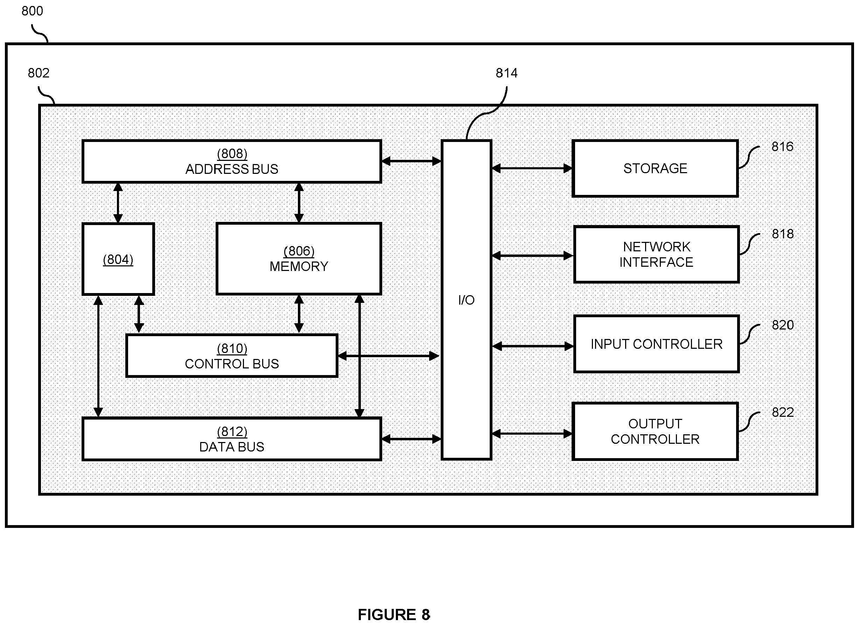

Figure 8 shows an example computing system for carrying out the methods of the present disclosure. Specifically, Figure 8 shows a block diagram of an embodiment of a computing system according to example embodiments of the present disclosure.

Computing system 800 can be configured to perform any of the operations disclosed herein such as, for example, any of the operations discussed with reference to the functional units described in relation to Figure 1 or the steps discussed with reference to Figures 6 and 7. Computing system includes one or more computing device(s) 802. The one or more computing device(s) 802 of computing system 800 comprise one or more processors 804 and memory 806. One or more processors 804 can be any general purpose processor(s) configured to execute a set of instructions. For example, one or more processors 804 can be one or more general-purpose processors, one or more field programmable gate array (FPGA), and/or one or more application specific integrated circuits (ASIC). In one embodiment, one or more processors 804 include one processor. Alternatively, one or more processors 804 include a plurality of processors that are operatively connected. One or more processors 804 are communicatively coupled to memory 806 via address bus 808, control bus 810, and data bus 812. Memory 806 can be a random access memory (RAM), a read only memory (ROM), a persistent storage device such as a hard drive, an erasable programmable read only memory (EPROM), and/or the like. The one or more computing device(s) 802 further comprise I/O interface 814 communicatively coupled to address bus 808, control bus 810, and data bus 812.

Memory 806 can store information that can be accessed by one or more processors 804. For instance, memory 806 (e.g., one or more non-transitory computer-readable storage mediums, memory devices) can include computer-readable instructions (not shown) that can be executed by one or more processors 804. The computer-readable instructions can be software written in any suitable programming language or can be implemented in hardware. Additionally, or alternatively, the computer-readable instructions can be executed in logically and/or virtually separate threads on one or more processors 804. For example, memory 806 can store instructions (not shown) that when executed by one or more processors 804 cause one or more processors 804 to perform operations such as any of the operations and functions for which computing system 800 is configured, as described herein. In addition, or alternatively, memory 806 can store data (not shown) that can be obtained, received, accessed, written, manipulated, created, and/or stored. In some implementations, the one or more computing device(s) 802 can obtain from and/or store data in one or more memory device(s) that are remote from the computing system 800.

Computing system 800 further comprises storage unit 816, network interface 818, input controller 820, and output controller 822. Storage unit 816, network interface 818, input controller 820, and output controller 822 are communicatively coupled to the central control unit (i.e., the memory 806, the address bus 808, the control bus 810, and the data bus 812) via I/O interface 814.

Storage unit 816 is a computer readable medium, preferably a non-transitory computer readable medium, comprising one or more programs, the one or more programs comprising instructions which when executed by the one or more processors 804 cause computing system 800 to perform the method steps of the present disclosure. Alternatively, storage unit 816 is a transitory computer readable medium. Storage unit 816 can be a persistent storage device such as a hard drive, a cloud storage device, or any other appropriate storage device.

Network interface 818 can be a Wi-Fi module, a network interface card, a Bluetooth module, and/or any other suitable wired or wireless communication device. In an embodiment, network interface 818 is configured to connect to a network such as a local area network (LAN), or a wide area network (WAN), the Internet, or an intranet.

A wearable device suitable for executing the above described aspects of the present disclosure is shown in Figure 9. In one example, the wearable device is a device for cuff-less blood pressure estimation.

Figure 9 shows a wearable device 900 comprising a computing environment 902, a screen 904, and a band 906. The computing environment 902 comprises a first sensor 908, a second sensor 910, one or more processors 912 (i.e., the processing unit), and a memory 914. It is to be noted that the computing environment 902 shown in Figure 9 is deliberately simplified and in some embodiments may more closely resemble the computing system 800 shown in Figure 8. It should be noted that both the screen 904 (i.e., the output unit) and the band 906 are optional.

In one example, the first sensor 908 comprises a photoplethysmogram (PPG) sensor and the second sensor 910 comprises an electrocardiogram (ECG) sensor. In examples embodiments, the computing environment 902 comprises only one of the first sensor 908 or the second sensor 910. In yet a further example, the first sensor 908 and/or the second sensor 910 are separate from the computing environment 902 and accessible by means of a separate interface or controller (such as input controller 820 shown in Figure 8).

The first sensor 908 and the second sensor 910 are configured to obtain measurements or signals, e.g., a PPG signal and an ECG sensor, which can be received by one or more processors 912. The one or more processors 912 can be any general-purpose processor(s) configured to execute a set of instructions. For example, the one or more processors 912 can be one or more general-purpose processors, one or more field programmable gate array (FPGA), and/or one or more application specific integrated circuits (ASIC). In one example, the one or more processors 912 include one processor. Alternatively, the one or more processors 912 include a plurality of processors that are operatively connected. The one or more processors 912 are communicatively coupled to the memory 914 via an address bus, a control bus, and a data bus (not shown).

The memory 914 can be a random-access memory (RAM), a read-only memory (ROM), a persistent storage device such as a hard drive, an erasable programmable read-only memory (EPROM), and/or the like. The memory 914 can store information that can be accessed by the one or more processors 912. For instance, the memory 914 (e.g., one or more non-transitory computer-readable storage mediums, memory devices) can include computer-readable instructions (not shown) that can be executed by the one or more processors 912. The computer-readable instructions can be software written in any suitable programming language or can be implemented in hardware. Additionally, or alternatively, the computer-readable instructions can be executed in logically and/or virtually separate threads on the one or more processors 912. For example, the memory 914 can store instructions (not shown) that when executed by the one or more processors 912 cause the one or more processors 912 to perform operations such as any of the operations and functions for which wearable the computing device 900 is configured, as described herein. In addition, or alternatively, the memory 914 can store data (not shown) that can be obtained, received, accessed, written, manipulated, created, and/or stored. The data can include, for instance, the data and/or information described herein in relation to Figures 1 to 5. In some implementations, the wearable computing device 900 can obtain from and/or store data in one or more memory device(s) that are remote from the wearable computing device 900.

Figures 8 and 9 illustrate example systems 800, 900 that can be used to implement aspects of the present disclosure. These computing systems are not intended to be limiting and other computing systems, and various modifications of example systems 800, 900, can be used as well. Computing tasks discussed herein can instead be performed remote from the respective system, or vice versa. Such configurations can be implemented without deviating from the scope of the present disclosure. The use of computer-based systems allows for a great variety of possible configurations, combinations, and divisions of tasks and functionality between and among components. Computer-implemented operations can be performed on a single component or across multiple components. Computer-implemented tasks and/or operations can be performed sequentially or in parallel. Data and instructions can be stored in a single memory device or across multiple memory devices.

It is to be noted that the foregoing description is merely used for enabling the skilled person to understand the present disclosure, without any intention to limit the applicability of the present disclosure to other embodiments which could be readily understood and/or envisaged by the reader. In particular, whilst the present disclosure is primarily directed to the translation of source (stochastic) biological signal to a target (stochastic) biological signal, the skilled person will appreciate that the present disclosure is applicable to other areas of technology involving the translation of stochastic signals having a common or related origin.

In the present disclosure, references to items in the singular should be understood to include items in the plural, and vice versa, unless explicitly stated otherwise or clear from the context. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth. The use of any and all examples, or exemplary language ("e.g.," "such as," "including," or the like) provided herein, is intended merely to better illuminate the embodiments and does not pose a limitation on the scope of the embodiments or the claims.

The systems and methods of the present disclosure are applicable to any suitable programming language or environment, including but not limited to Java, C, C++, any suitable assembly language, Python, C#, JavaScript, Ruby, PHP, and the like.

Some aspects described herein may relate to a computer storage product with a non-transitory computer-readable medium (also can be referred to as a non-transitory processor-readable medium) having instructions or computer code thereon for performing various computer implemented operations. The computer-readable medium (or processor readable medium) is non-transitory in the sense that it does not include transitory propagating signals per se (e.g., a propagating electromagnetic wave carrying information on a transmission medium such as space or a cable). The media and computer code (also can be referred to as code) may be those designed and constructed for the specific purpose or purposes. Examples of non-transitory computer-readable media include, but are not limited to, magnetic storage media such as hard disks, floppy disks, and magnetic tape; optical storage media such as Compact Disc/Digital Video Discs (CD/DVDs), Compact Disc-Read Only Memories (CD ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a transitory computer program product, which can include, for example, the instructions and/or computer code discussed herein.

Some processes/methods described herein can be performed by software (executed on hardware), hardware, or a combination thereof. Hardware modules include, for example, a general-purpose processor, a field programmable gate array (FPGA), and/or an application specific integrated circuit (ASIC). Software modules (executed on hardware) can be expressed in a variety of software languages (e.g., computer code), including C, C++, Java, Ruby, Visual Basic, Python, and/or other object-oriented, procedural, or other programming language and development tools. Examples of computer code include, but are not limited to, micro-code or micro-instructions, machine instructions, such as produced by a compiler, code used to produce a web service, and files containing higher-level instructions that are executed by a computer using an interpreter. For example, the processes/methods can be implemented using imperative programming languages (e.g., C, Fortran, etc.), functional programming languages (Haskell, Erlang, etc.), logical programming languages (e.g., Prolog), object-oriented programming languages (e.g., Java, C++, etc.) or other suitable programming languages and/or development tools. Additional examples of computer code include, but are not limited to, control signals, encrypted code, and compressed code.

### ASPECTS OF THE DISCLOSURE

Non-limiting aspects of the disclosure are set out in the following numbered statements.
1. A device for cuff-less blood pressure estimation, the device comprising:
   a photoplethysmogram (PPG) sensor configured to obtain PPG measurements from a user of the device;
   a processing unit comprising one or more processors configured to:
      obtain, from the PPG sensor, a PPG signal;
      decompose the PPG signal into one or more source components, the one or more source components comprising at least an AC component of the PPG signal;
      map, using one or more trained machine learning models, the one or more source components to a target AC component and a target DC component; and
      generate a transformed target AC component from the target AC component such that a first average value determined from the transformed target AC component matches a second average value determined from the target DC component, wherein the transformed target AC component corresponds to an approximate arterial blood pressure (ABP) signal for the user; and
   an output unit configured to provide one or more features of the transformed target AC component for review by the user.
2. The device of statement 1 wherein the one or more source components comprise a source AC component generated using a continuous wavelet transform (CWT) and a source DC component generated using a Fourier transform.
3. The device of statement 2 wherein the one or more processors of the processing unit are further configured to:
   inverse transform the target AC component using an inverse CWT transform and the target DC component using a phase reconstruction algorithm such that the transformed target AC component is generated from inverse transformed representations of the target AC component and the target DC component.
4. The device of statement 2 or statement 3 wherein the source AC component is mapped to the target AC component using a first convolutional neural network of the one or more trained machine learning models and the source DC component is mapped to the target DC component using a second convolutional neural network of the one or more trained machine learning models.
5. The device of any preceding statement wherein the one or more source components are mapped to the target AC component and the target DC component using a convolutional neural network of the one or more trained machine learning models having a shared encoder and at least two decoders each of which being associated with a respective one of the target AC component or the target DC component.
6. The device of any preceding statement wherein the one or more features provided by the output unit include one or both of an estimated diastolic blood pressure and an estimated systolic blood pressure estimated from one or more peaks and one or more troughs of the transformed target AC component.
7. An electronically-implemented method for translation of signals having shared origin, the method comprising:
   decomposing a source signal into a first source component and a second source component, wherein the first source component encodes oscillatory behavior of the source signal and the second source component encodes steady-state behavior of the source signal;
   mapping, using one or more trained models, the first source component and the second source component to a first target component and a second target component respectively, wherein the first target component encodes estimated oscillatory behavior of a target signal and the second target component encodes estimated steady-state behavior of the target signal; and
   reconstructing the target signal from the first target component and the second target component.
8. The electronically-implemented method of statement 7 wherein the source signal is decomposed into the first source component and the second source component using a first transformation process and a second transformation process respectively.
9. The electronically-implemented method of statement 8 wherein the first transformation process is a continuous wavelet transform and the second transformation process is a Fourier transform.
10. The electronically-implemented method of statement 8 or statement 9 further comprising, prior to the step of generating the target signal:
   inverse transforming the first target component and the second target component using a first inverse transformation process and a second inverse transformation process respectively, wherein the first inverse transformation process and the second inverse transformation process are inverses of the first transformation process and the second transformation process respectively.
11. The electronically-implemented method of any of statements 7 to 10 wherein the first source component is mapped to the first target component using a first trained model of the one or more trained models and the second source component is mapped to the second target component using a second trained model of the one or more trained models.
12. The electronically-implemented method of any of statements 7 to 11 wherein the target signal comprises the first target component transformed such that a first average value determined from the target signal matches a second average value determined from the second target component.
13. The electronically-implemented method of any of statements 7 to 12 wherein the source signal is a photoplethysmogram (PPG) signal and the target signal is an arterial blood pressure (ABP) signal.
14. A computer program comprising instructions which, when executed by a device comprising one or more processors, cause the device to carry out the steps of:
   obtaining a source stochastic signal;
   transforming, using a first transformation process, the source stochastic signal to a first complex map, wherein the first complex map encodes an oscillatory behavior of the source stochastic signal;
   transforming, using a second transformation process, the source stochastic signal to a second complex map, wherein the second complex map encodes a steady-state behavior of the source stochastic signal;
   mapping, using one or more neural networks, the first complex map and the second complex map to a third complex map and a fourth complex map respectively, wherein the third complex map and the fourth complex map are associated with a target stochastic signal;
   transforming, using a first inverse transformation process associated with the first transformation process, the third complex map to a first component of the target stochastic signal, wherein the first component of the target stochastic signal represents the oscillatory behavior of the target stochastic signal;
   transforming, using a second inverse transformation process associated with the second transformation process, the fourth complex map to a second component of the target stochastic signal, wherein the second component of the target stochastic signal represents the steady-state behavior of the target stochastic signal; and
   reconstructing the target stochastic signal from the first component of the target stochastic signal and the second component of the target stochastic signal.
15. The computer program of statement 14 wherein the source stochastic signal is a photoplethysmogram (PPG) signal and the target stochastic signal is an arterial blood pressure (ABP) signal.
16. The computer program of statement 14 or statement 15 wherein the first complex map is a scalogram and the second complex map is a spectrogram.
17. The computer program of statement 16 wherein the first transformation process is a continuous wavelet transform (CWT) and the second transformation process is a Fourier transform.
18. The computer program of statement 17 wherein the first inverse transformation process is estimated using an inverse CWT and the second inverse transformation process is estimated using a phase reconstruction algorithm.
19. The computer program of any of statements 14 to 18 wherein the first complex map is mapped to the third complex map using a first trained neural network and the second complex map is mapped to the third complex map using a second trained neural network.
20. The computer program of any of statements 14 to 19 wherein the instructions, when executed by the device, further cause the device to carry out the step of:
   outputting the target stochastic signal.

## Claims

1. A device for cuff-less blood pressure estimation, the device comprising:
a photoplethysmogram (PPG) sensor configured to obtain PPG measurements from a user of the device;
a processing unit comprising one or more processors configured to:
obtain, from the PPG sensor, a PPG signal;
decompose the PPG signal into one or more source components, the one or more source components comprising at least an AC component of the PPG signal;
map, using one or more trained machine learning models, the one or more source components to a target AC component and a target DC component; and
generate a transformed target AC component from the target AC component such that a first average value determined from the transformed target AC component matches a second average value determined from the target DC component, wherein the transformed target AC component corresponds to an approximate arterial blood pressure (ABP) signal for the user; and
an output unit configured to provide one or more features of the transformed target AC component for review by the user.

2. The device of claim 1 wherein the one or more source components comprise a source AC component generated using a continuous wavelet transform (CWT) and a source DC component generated using a Fourier transform.

3. The device of claim 2 wherein the one or more processors of the processing unit are further configured to:
inverse transform the target AC component using an inverse CWT transform and the target DC component using a phase reconstruction algorithm such that the transformed target AC component is generated from inverse transformed representations of the target AC component and the target DC component.

4. The device of claim 2 or claim 3 wherein the source AC component is mapped to the target AC component using a first convolutional neural network of the one or more trained machine learning models and the source DC component is mapped to the target DC component using a second convolutional neural network of the one or more trained machine learning models.

5. The device of any preceding claim wherein the one or more source components are mapped to the target AC component and the target DC component using a convolutional neural network of the one or more trained machine learning models having a shared encoder and at least two decoders each of which being associated with a respective one of the target AC component or the target DC component.

6. The device of any preceding claim wherein the one or more features provided by the output unit include one or both of an estimated diastolic blood pressure and an estimated systolic blood pressure estimated from one or more peaks and one or more troughs of the transformed target AC component.

7. An electronically-implemented method for translation of signals having shared origin, the method comprising:
decomposing a photoplethysmogram (PPG) signal into a first PPG component and a second PPG component, wherein the first PPG component encodes oscillatory behavior of the PPG signal and the second PPG component encodes steady-state behavior of the PPG signal;
mapping, using one or more trained models, the first PPG component and the second PPG component to a first arterial blood pressure (ABP) component and a second ABP component respectively, wherein the first ABP component encodes estimated oscillatory behavior of a ABP signal and the second ABP component encodes estimated steady-state behavior of the ABP signal; and
reconstructing the ABP signal from the first ABP component and the second ABP component.

8. The electronically-implemented method of claim 7 wherein the PPG signal is decomposed into the first PPG component and the second PPG component using a first transformation process and a second transformation process respectively.

9. The electronically-implemented method of claim 8 wherein the first transformation process is a continuous wavelet transform and the second transformation process is a Fourier transform.

10. The electronically-implemented method of claim 8 or claim 9 further comprising, prior to the step of generating the ABP signal:
inverse transforming the first ABP component and the second ABP component using a first inverse transformation process and a second inverse transformation process respectively, wherein the first inverse transformation process and the second inverse transformation process are inverses of the first transformation process and the second transformation process respectively.

11. The electronically-implemented method of any preceding claim wherein the first PPG component is mapped to the first ABP component using a first trained model of the one or more trained models and the second PPG component is mapped to the second ABP component using a second trained model of the one or more trained models.

12. The electronically-implemented method of any preceding claim wherein the ABP signal comprises the first ABP component transformed such that a first average value determined from the ABP signal matches a second average value determined from the second ABP component.

13. The electronically-implemented method of any preceding claim further comprising:
outputting the ABP signal.

14. A computer readable medium storing instructions which, when executed by a device comprising one or more processors, cause the device to carry out the steps of:
obtaining a photoplethysmogram (PPG) signal;
transforming, using a first transformation process, the PPG signal to a first complex map, wherein the first complex map encodes an oscillatory behavior of the PPG signal;
transforming, using a second transformation process, the PPG signal to a second complex map, wherein the second complex map encodes a steady-state behavior of the PPG signal;
mapping, using one or more neural networks, the first complex map and the second complex map to a third complex map and a fourth complex map respectively, wherein the third complex map and the fourth complex map are associated with a arterial blood pressure (ABP) signal;
transforming, using a first inverse transformation process associated with the first transformation process, the third complex map to a first component of the ABP signal, wherein the first component of the ABP signal represents the oscillatory behavior of the ABP signal;
transforming, using a second inverse transformation process associated with the second transformation process, the fourth complex map to a second component of the ABP signal, wherein the second component of the ABP signal represents the steady-state behavior of the ABP signal; and
reconstructing the ABP signal from the first component of the ABP signal and the second component of the ABP signal.

15. The computer readable medium of claim 14 wherein the first complex map is a scalogram and the second complex map is a spectrogram.
